# EUROPEAN PATENT APPLICATION

(11) **EP 1 321 884 A2**
(43) Date of publication of application: **25.06.2003**
(21) Application number: 02028068.1
(22) Date of filing: 17.12.2002
(51) Int. Cl.: G06F 19/00

(54) **Endoscope image filing system**

(30) Priority: 21.12.2001 JP 2001390361
(71) Applicant: Olympus Optical Co., Ltd., Tokyo 151-0072 (JP)
(72) Inventor: Ohishi, Hiroko, Hachioji-shi, Tokyo (JP); Watai, Makoto, Northport, New York 11768 (US); Shibata, Hiroyuki, Yokohama-shi, Kanagawa (US)
(74) Representative: von Hellfeld, Axel, Dr. Dipl.-Phys.

(57) **Abstract**

A patient information deleting setting screen is provided to set a condition to delete unnecessary patient information, and a term for which patient information is registered but the endoscope examination has not been performed is set as a deleting condition. The deleting condition is recorded and, upon starting an endoscope image filing system next time, the patient information under the deleting condition is automatically deleted. Thus, the occupation of a database due to the storage of the unnecessary patient information is prevented and the operability is improved to search the patient information for a short time.

## Description

### BACKGROUND OF THE INVENTION

### 1. Field of the Invention

The present invention relates to an endoscope image filing system for recording an endoscope image.

### 2. Description of the Related Art

Conventionally, an endoscope device comprising image pick-up means in an endoscope for observing a subject portion such as the body cavity by inserting a long insertion portion is used to display on a monitor a subject portion image, i.e., an endoscope image, which is picked up by the image pick-up means.

In recent years, an endoscope image filing system is widely used, in which an (endoscope) image filing device for recording the endoscope image is connected to the endoscope device.

In the endoscope image filing system, the endoscope image displayed on the monitor is recorded to the endoscope image filing device by pressing an endoscope switch, that is, a release switch incorporated in the endoscope device.

By connecting a hospital information system to a network, the endoscope image filing system records and searches not only the endoscope image but also various information on an endoscope examination including patient information such as a name, age, and sex of a patient, endoscope examination reserved information such as a reserved date of the endoscope examination, reserved time, and the type of examination, and examination information such as doctor remarks of a recorded endoscope image, examined date of the endoscope examination, and examined time. Further, the endoscope image filing system registers the patient information inputted by using tools other than the endoscope.

However, the image filing device in the conventional endoscope image filing system registers unnecessary patient information other than the patient information without using the endoscope and the patient information having no relationship with the endoscope examination which is erroneously inputted by a user. Therefore, the unnecessary patient information occupies a database of the image filing device.

Further, since a large amount of unnecessary data is registered, when the user searches the patient information, the data search requires a long time.

### SUMMARY OF THE INVENTION

Accordingly, it is an object of the present invention to provide an endoscope image filing device to improve the operability when searching information on patients registered in a database.

An endoscope image filing system comprises: an image filing device for recording an endoscope image and forming a database in which patient information is registered; registering means for registering the patient information; deleting condition storing means for storing a deleting condition for deleting the patient information registered by the registering means; and patient information deleting means for deleting the registered patient information based on the registering condition stored by the deleting condition storing means.

Thus, the patient information is deleted under an arbitrary condition. For example, the patient information which is not registered is deleted. When searching the patient information from the database, the unnecessary patient information is not found and the amount of the registered information in the database is not unnecessarily increased. Accordingly, the operability can be improved.

### BRIEF DESCRIPTION OF THE DRAWINGS

Fig. 1 is a diagram showing the entire structure of an endoscope image filing system according to an embodiment of the present invention;
Fig. 2 is a block diagram for explaining the hardware structure of an image filing device according to the embodiment;
Fig. 3 is an explanatory diagram of the outline of the screen structure of the image filing device according to the embodiment;
Fig. 4 is a flowchart for explaining the entire operation of the image filing device according to the embodiment;
Fig. 5 is a flowchart when automatically registering new patient information from an in-hospital information system connected to the endoscope image filing system via an external network according to the embodiment;
Fig. 6 is a diagram showing a display example of a schedule list screen according to the embodiment;
Fig. 7 is a diagram showing a display example of a patient information list screen according to the embodiment;
Fig. 8 is a diagram showing a display example of a patient information editing screen according to the embodiment;
Fig. 9 is a flowchart for setting the deletion of the patient information according to the embodiment;
Fig. 10 is a diagram showing a display example of a patient information deletion setting screen according to the embodiment;
Fig. 11 is a flowchart for deleting the patient information according to the embodiment; and
Fig. 12 is a flowchart for setting the deletion of the patient information according to a modification of the embodiment.

### DESCRIPTION OF THE PREFERRED EMBODIMENTS

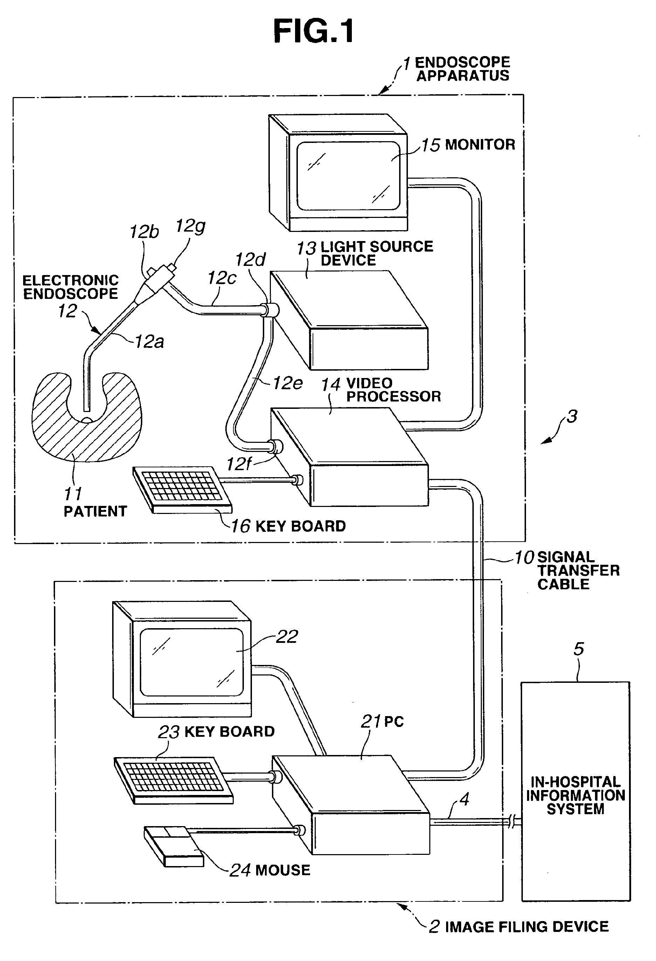

An embodiment of the present invention will be described with reference to Figs. 1 to 12.

Referring to Fig. 1, an endoscope image filing system 3 comprises an endoscope device 1 and an endoscope image filing device (hereinafter, abbreviated to an image filing device) 2 connected to the endoscope device 1 via a signal transfer cable 10 according to the embodiment. The endoscope image filing system 3 (comprising the image filing device 2) is connected to an in-hospital information system 5 as an external system via a network cable 4, thereby receiving and transmitting information from/to the in-hospital information system 5.

The endoscope device 1 comprises an electronic endoscope (hereinafter, abbreviated to an endoscope for a simple description) 12 for endoscope examination, into which an insertion portion 12a is inserted in the body of a patient 11, a light source device 13 for supplying illumination light to the endoscope 12 by connecting a connector 12d at the end portion of a universal cable 12c projecting from an operation portion 12b of the endoscope 12, a video processor 14 for signal processing for generating a video signal (image signal) for an image pick-up device incorporated in the endoscope 12 by freely detachably being connected to a signal connector 12f at the end portion of a signal cable 12e projecting from a connector 12d, a monitor 15 for displaying an endoscope image which is picked up by the image pick-up device by being connected to the video processor 14 and by inputting the video signal, and a key board 16 connected to the video processor 14, for inputting data.

A release switch 12g is provided for the operation portion 12b in the endoscope 12. By operating the release switch 12g, a release instructing signal is transmitted to the image filing device 2 via the video processor 14 and the endoscope image is recorded to the image filing device 2.

The image filing device 2 comprises a personal computer (PC) 21, a monitor 22 for displaying the endoscope image and another information, and a key board 23 and a mouse 24 connected to the personal computer 21, for inputting data and an instruction command and for instructing the selection.

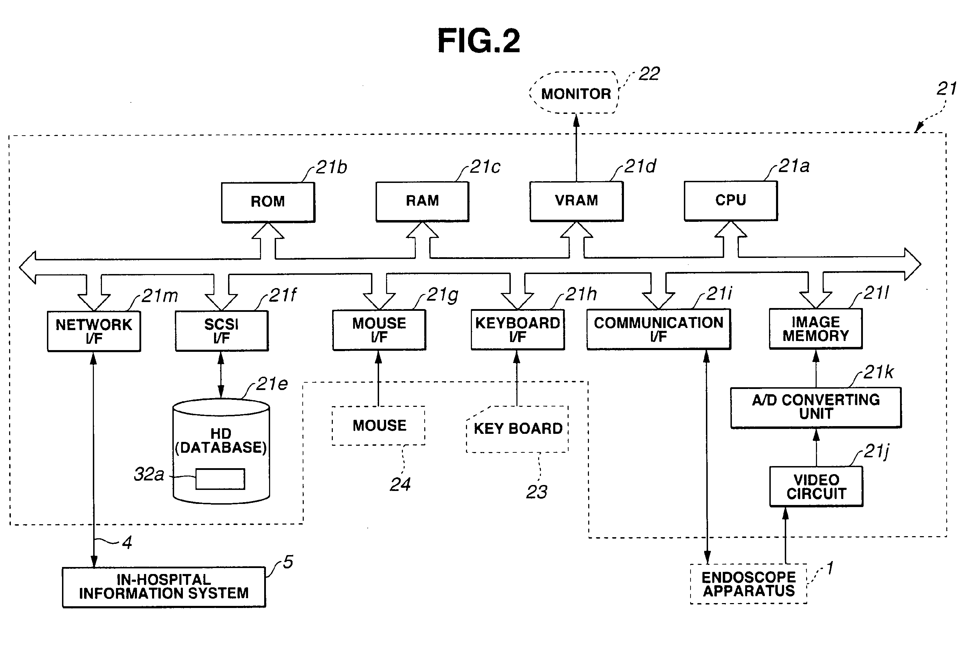

Referring to Fig. 2, a description is given respecting the internal structure of the personal computer 21 as a main portion of the image filing device 2.

The personal computer 21 comprises a CPU 21a as main control means for controlling portions of the personal computer 21, a ROM 21b for storing a program for operating the CPU 21a and information such as a display message to the monitor 22, a RAM 21c used as a temporarily storing area of various data and a working area of the CPU 21a, a VRAM 21d for temporarily storing image data outputted to the monitor 22, a hard disk 21e for storing the various data such as the image data and patient data as a database, a SCSI interface 21f for receiving and transmitting data from/to the hard disk 21e by a well-known SCSI system, a mouse interface 21g for detecting an input from the mouse 24, a key board interface 21h for detecting an input from the key board 23 for inputting a deleting condition for deleting the patient information and for inputting the registering data of the patient information, a communication interface 21i for receiving and transmitting the various data such as a release instruction to the endoscope device 1 in a well-known RS232C or USB system, a video circuit 21j as an interface circuit for inputting the image signal outputted from (the video processor 14) of the endoscope device 1, an A/D converting unit 21k for A/D converting the image signal inputted by the video circuit 21j, an image memory 211 for temporarily storing the image data outputted from the A/D converting unit 21k, and a network interface 21m for being connected to the in-hospital information system 5 via a network.

As above, the image filing device 2 mainly comprises the personal computer 21, displays the image data obtained by the endoscope device 1 on the monitor 22, stores the patient information in the hard disk 21e with a corresponding relationship therebetween, and uses the stored information for searching desired image data and the patient information as a database.

In the image filing device 2, an operator inputs the data or instruction through the key board 23 or the mouse 24 in accordance with various screens displayed on the monitor 22. The CPU 21a controls the components in accordance with the input data or instruction, thus to execute processing corresponding to the instruction. That is, the image filing device 2 executes various processing in accordance with the flow on the screen displayed on the monitor 22.

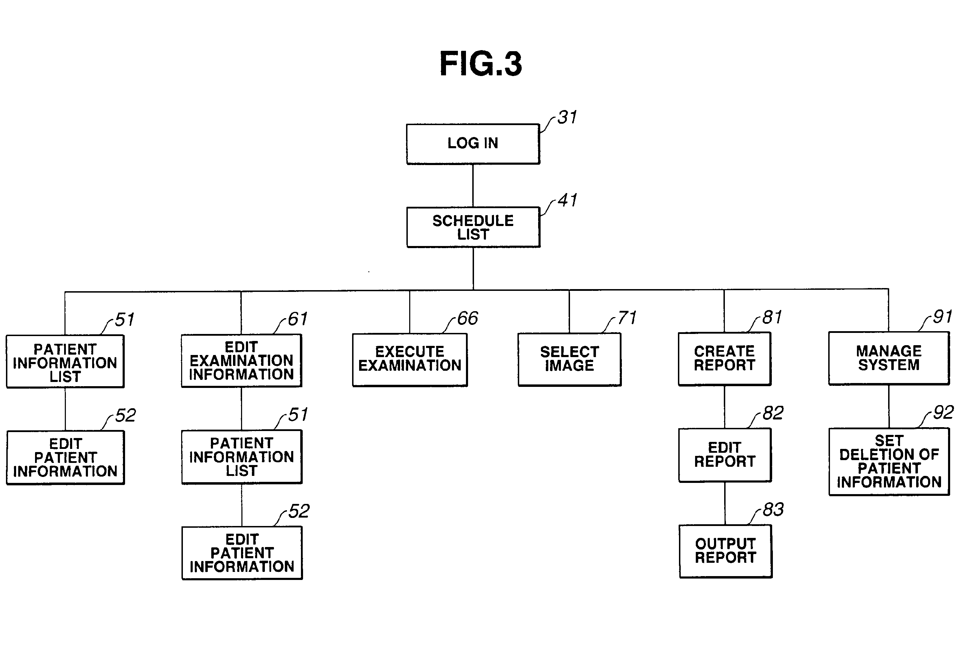

Hereinbelow, a description is given respecting the outline of the screen structure of the image filing device 2 with reference to Fig. 3.

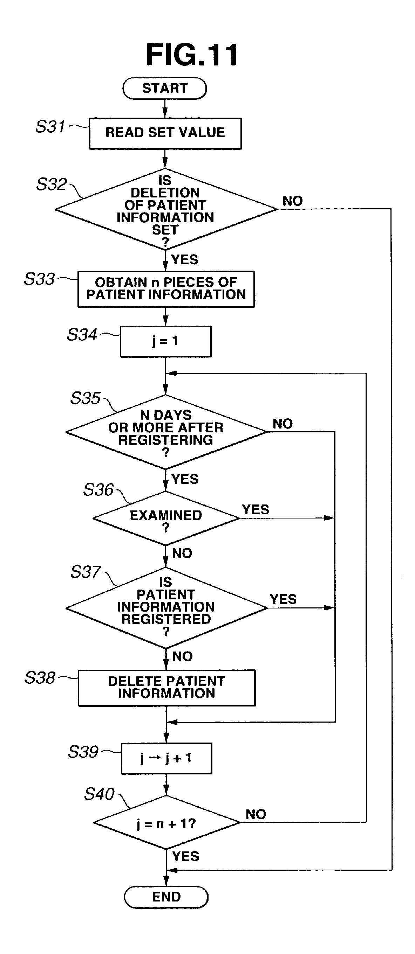

First, a log-in screen 31 for authenticating the operator is displayed upon starting the image filing device 2. The image filing device 2 is started according to the embodiment as will be described (with reference to Fig. 11). Then, the image filing device 2 executes the operation for deleting the patient information in accordance with a condition which has already been set and, thereafter, the log-in screen 31 is displayed.

The operator is authenticated on the log-in screen 31, thereby displaying a schedule list screen 41 for displaying a list of an examination schedule or the like.

On the schedule list screen 41, a patient list screen 51 for displaying a list of the patient information is called. On the patient list screen 51, the patient information is newly registered and a patient information screen 52 for editing the patient information which has already been registered is called.

Further, on the schedule list screen 41, the patient information is newly registered and the examination is reserved. Furthermore, on the schedule list screen 41, an examination information editing screen 61 for editing the examination information which has already been registered is called. On the examination information editing screen 61, the patient list screen 51 is called.

In addition, on the schedule list screen 41, the examination is executed by the connection to the endoscope device 1 and an examination executing screen 66 for capturing the image from the endoscope device 1 is called.

In addition, on the schedule list screen 41, an image selecting screen 71 for selecting an image based on an examination report created in the captured images is called.

In addition, on the schedule list screen 41, a report creating screen 81, which is one of screens for creating the examination report, is called. On the report creating screen 81, a report editing screen 82 for editing the report is called. The report editing screen 82 shifts to a report output screen 83 for printing the report.

In addition, on the schedule list screen 41, a system management screen 91 as a screen for inputting the operation for setting the endoscope image filing system 3 is called. The system management screen 91 shifts to a patient information deletion setting screen 92 for setting a condition for deleting the patient information registered in the database.

As will be described later, by setting the condition for deleting the unnecessary patient information on the patient information deletion setting screen 92, the information on the setting condition is recorded as a deleting condition in the hard disk 21e (the hard disk 21e constitutes the database and includes a deleting condition storing area 32a (refer to Fig. 2) for storing the deleting condition).

Upon starting the image filing device 2 next time, the information on the setting information is read from the deleting condition storing area 32a, the patient information registered in the database corresponding to the information is automatically deleted, the occupation of the database by the unnecessary patient information is prevented, and the unnecessary patient information is deleted so that the patient information is searched in a short time, thus to improve the operability.

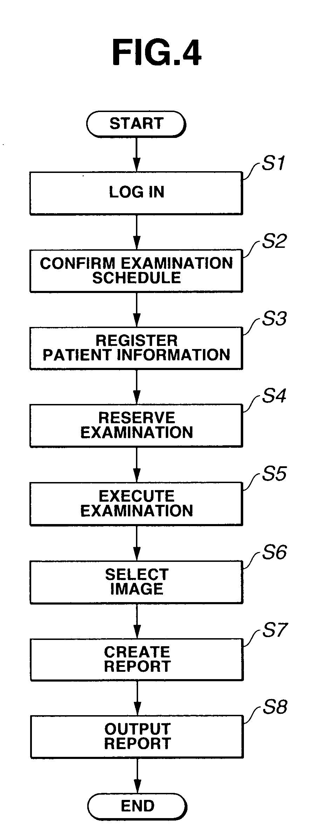

An example of the entire operation of the image filing device 2 will be described with reference to Fig. 4. Herein, reference numerals S1 to S8 denote processing steps.

First, the image filing device 2 is started and then the log-in screen 31 is displayed. Herein, the operator is authenticated and, in step S1, the log-in enables the operation for displaying the schedule list screen 41. In step S2, the examination schedule is confirmed.

Next, when the patient as an examination target is a new one, the patient list screen 51 and the patient information editing screen 52 are called and then, in step S3, the patient information is registered.

Subsequently, the examination information editing screen is called and, in step S4, the new examination reservation is inputted.

Further, the examination executing screen 66 is called and, in step S5, the examination is executed by the endoscope device 1 connected to the image filing device 2. Then, the image obtained by the endoscope device 1 is stored in the image filing device 2.

Next, the image selecting screen 71 is called. Processing in step S6 is performed to select an image referred by the examination report created from the images obtained by executing the examination.

Next, the report creating screen 81 is called. In step S7, the examination report is created. In step S8, the report is outputted (printed).

According to the embodiment, the patient information deletion setting screen 92 is set to delete the unnecessary patient information as will be described later. Thus, the unnecessary patient information can be deleted.

The above is an example of the entire flow of the rough operation.

Hereinbelow, the detailed structure and operation of the screens will be described according to the embodiment.

In the present specification, the appearing of the screen which is not displayed is expressed as "the screen is opened". Further, the ending of the operation of displaying the screen which has been displayed is expressed as "the screen is closed".

Further, in the drawings referred to in the present specification, the operation of displaying a message and the like in the drawings indicating the screen display uses English and partly uses German. However, it may be Japanese or other languages and drawings.

The button is a specific area on the screen which is indicated by a graphic or character for properly indicating a button function. A stereoscopic view is expressed by appropriately displaying the shading in the specific area and the shape of a press button switch is expressed on the screen. By clicking the button, a cursor of the mouse 24 is moved in the area of the button on the screen and the mouse 24 is clicked. By clicking the button on the screen, functions corresponding to the buttons are executed.

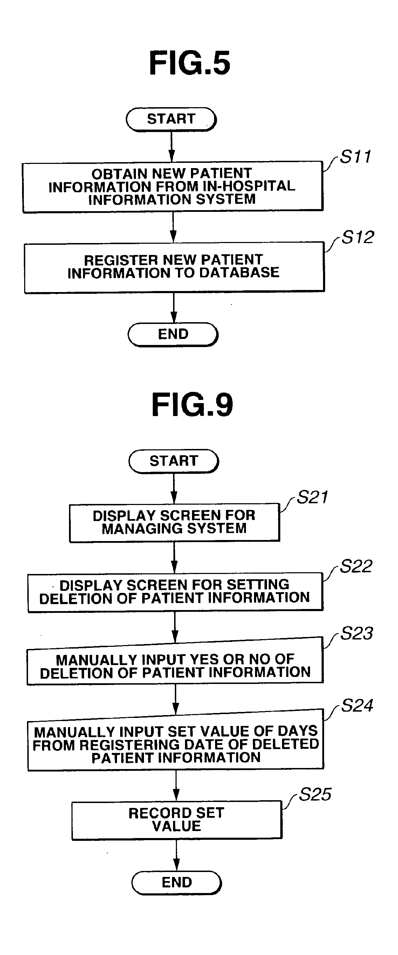

Fig. 5 is a flowchart for explaining the operation for automatically registering the new patient information from the in-hospital information system 5 connected to the endoscope image filing system 4 via the network cable 4.

The endoscope image filing system 3 is started and then the operator is authenticated on the log-in screen 31. In step S11, the CPU 21a in the image filing device 2 obtains a new patient information, which has never been registered in the database constituted by the hard disk 21e in the image filing device 2, from (the endoscope image filing device 2 or the endoscope image filing system 3 of) the in-hospital information system 5. In step S12, the new patient information is registered in the database (of the endoscope image filing device 2) in the endoscope image filing system 3.

In this case, the new patient information may be obtained from the in-hospital information system 5 and be registered in the database of the endoscope image filing device 2 at the timing to start or end the endoscope image filing system 3 or at the user's setting timing.

Upon registering the new patient, not only the patient information but also the examination result at another section registered in the in-hospital information system 5 may be registered. The operation for registering the new patient information can be performed by the key board 23 in the image filing device 2.

The examination reservation information can be registered in the in-hospital information system 5. In this case, upon registering the new patient, the examination reservation information may be registered there and may be registered in the endoscope image filing system 3.

Upon registering the new patient, the endoscope examination for registering the examination reservation information in the endoscope image filing system 3 from the in-hospital information system 5 is executed and the report is created. Then, chart information may be registered in the in-hospital information system 5 from the endoscope image filing system 3.

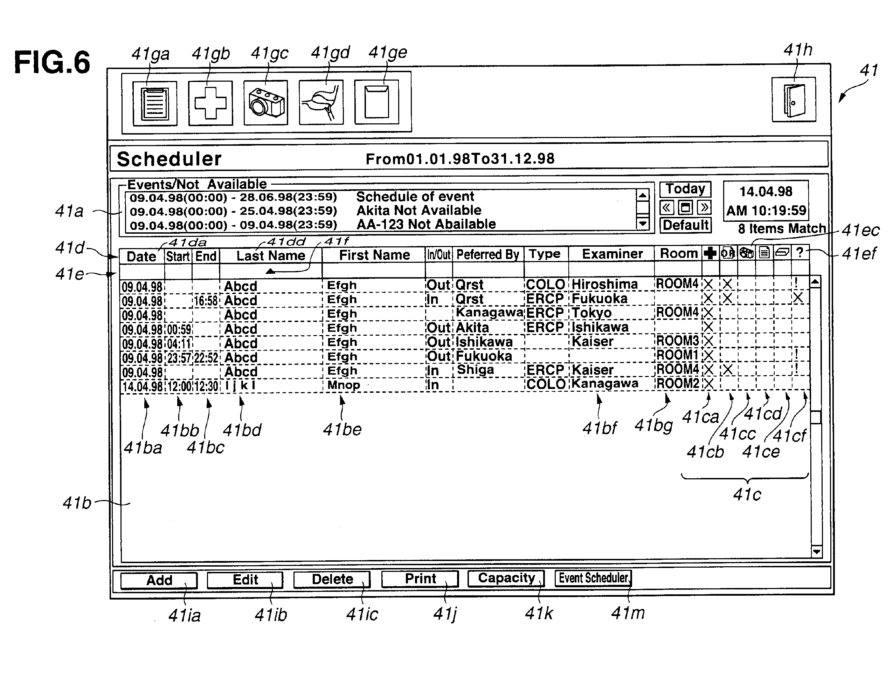

Fig. 6 is a display example of the schedule list screen 41.

The schedule list screen 41 comprises a resource schedule list display area 41a for displaying a schedule list of an examination resource and an examination information list display area 41b for displaying the list of the examination information.

One piece of the examination information is referred to as an examination information record in the present specification.

A first line of the examination information list display area 41b displays a caption of each data item constituting the examination information record.

The examination information record is displayed starting from a third line of the examination information list display area 41b. One examination information record is displayed in one line of the examination information list display area 41b.

The data item constituting the examination information record displayed at the examination information list display area 41b includes an (endoscope) examination date 41ba, an examination start time 41bb, an end time 41bc, a family name of the patient 41bd, a first name of the patient 41be, a name of an examining operator 41bf, a name of a used examination room 41bg, and the like.

The examination information display area 41b comprises an examining status display column 41c for displaying a state such as a progressing state of the examining operation.

An examining status display column 41ca constituting the examining status display column 41c displays whether or not all the input necessary items of the patient information as the examination target and all the input necessary items of the examination information have already been inputted. When all the input necessary items have already been inputted, a sign "×" is displayed and, in other cases, the examining status display column 41ca is blank.

An examining status display column 41cb displays whether or not the date at which an examination agreement received from the patient is inputted. When the examination has already been executed, the number pf pieces of sheets of the image recorded in the examination is displayed. When the examination has not been executed, the examining status display column 41cb is blank. Herein, the examination indicated by a number in an examining status display column 41cc means that the image of the subject portion of the patient has already been obtained from the examination.

An examining status display column 41cd displays whether or not the examination report has already been created. For example, when the examination report has already been created, a sign "×" is displayed. When the examination report has not been created, the examining status display column 41cd is blank.

An examining status display column 41ce displays whether or not an accounting code has already been inputted. When the accounting code has already been inputted, a sign "×" is displayed. When the accounting code has not been inputted, the examining status display column 41ce is blank.

An examining status display column 41cf displays a biopsy examining result when a biopsy is performed, for example, by taking the anatomy in the endoscope examination. When the biopsy examining result is obtained, a sign "×" is displayed. When the biopsy examining result is not obtained, a sign "!" is displayed. When the endoscope examination is performed but the biopsy is not performed, a sign "o" is displayed. When the endoscope examination is not performed, the examining status display column 41cf is blank.

The examination information list display area 41b displays not only all the examination information records but also the examination information record in which the user is interested by narrowing.

At the first line of the examination information list display area 41b, i.e., in the line for displaying each name of the data item, etc., filter buttons 41d as buttons for calling the screen for setting a narrowing condition to the data item are arranged.

Each filter button 41d is convex when the narrowing condition is not set to the corresponding data item.

The convex button indicates the shading to be embossed on the screen. A concave button indicates the shading to be dented on the screen.

The convex filter button 41d is clicked and then a narrowing condition setting screen as a screen for setting the narrowing condition to the corresponding data item, which will be described later, is opened to enable the input of the narrowing condition. Herein, the examination information list display area 41b displays only the examination information record which satisfies the narrowing condition with the narrowing condition inputted.

The filter button 41d becomes concave, corresponding to the data item to which the narrowing condition is set.

When a plurality of concave filter buttons 41d exist, the examination information records are narrowed and displayed under an AND condition of the narrowing conditions set by the filter buttons 41d.

The concave filter button 41d is clicked, then, the narrowing conditions set to the corresponding data item is canceled, and the filter button 41d becomes convex.

For example, a filter button 41da at the position corresponding to the examination date 41ba is clicked and the narrowing condition setting screen is called to set the narrowing conditions to the examination date 41ba.

A filter button 41dd is clicked at the position corresponding to the family name of the patient 41bd and the narrowing condition setting screen is called to set the conditions for narrowing the family names of the patients.

The examination information list display area 41b shown in Fig. 6 rearranges the order of the examination information records corresponding to the designated data items. A sort button 41e is arranged to rearrange the examination information records in the ascending or descending order corresponding to the data item at the second line in the examination information display area 41.

The sort button 41e is clicked and the examination information records are rearranged, e.g., in the ascending order corresponding to the data item at the sort button 41e, and a sign indicating the rearrangement in the ascending order, e.g., an up arrow 41f(▲) is displayed on the sort button 41e. The sort button 41e is clicked again and the examination information records are rearranged in the descending order and the up arrow 41f(▲) is changed to a down arrow (▼). Subsequently, the ascending order and the descending order are switched by clicking the sort button 41e.

In the examination information list display area 41b shown in Fig. 6, a filter button 41ec is clicked corresponding to the examining status display column 41cc indicating whether or not the examination is completed and, then, a narrowing condition setting screen is called to set whether or not the examination is completed as the narrowing condition.

In the examination information list display area 41b shown in Fig. 6, a filter button 41ef is clicked corresponding to the examining status display column 41cf indicating the biopsy examining result and the narrowing condition setting screen is called to set the biopsy examining result as the narrowing condition.

The schedule list screen 41 shown in Fig. 6 includes on the top, a button 41ga for calling the patient information list screen 51 by the click operation, a button 41gb for calling the examination information editing screen 61 by the click operation, a button 41gc for calling the examination executing screen 66 by the click operation, a button 41gd for calling the image selecting screen 71 by the click operation, and a button 41ge for calling the report creating screen 81 by the click operation.

On the upper right of the schedule list screen 41, an end button 41h is arranged to end the operation of the image filing device 2 by the click operation.

Further, the schedule list screen 41 includes on the bottom, a new button 41ia for calling the examination information editing screen 61 by the click operation upon newly reserving the examination, an edit button 41ib for calling the examination information editing screen 61 by the selecting and clicking the examination information record in the examination information list display area 41b upon editing the recorded examination information record, a delete button 41ic for deleting the selected examination information record by selecting and clicking the examination information record in the examination information list display area 41b, a print button 41j for printing out the examination information list by the click operation, a button 41k which is clicked upon displaying the working status of the examination room, and a button 41m which is clicked upon newly registering and editing the resource schedule record.

An examination room working status display screen is called by clicking the button 41k.

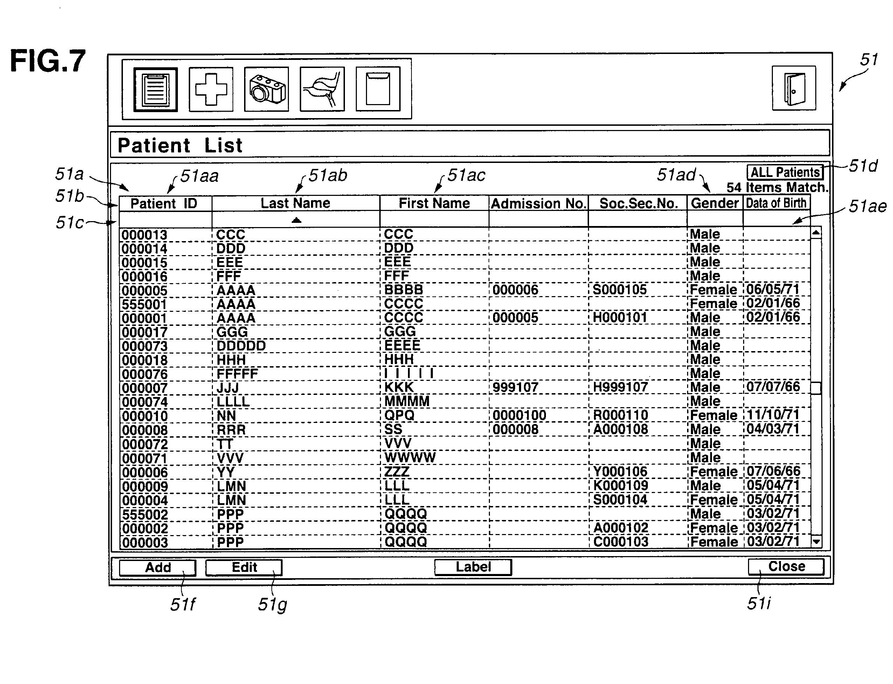

Fig. 7 is an example of a display screen of the patient list screen 51.

The patient list screen 51 includes a patient list display area 51a for displaying a list of the patient information. In the present specification, the patient information of one patient is conveniently called a patient information record. The patient information record is displayed at a line subsequent to the third line of the patient list display area 51a, and the one patient information record is corresponded to one line of the patient list display area 51a.

The patient information record displayed in the patient list display area 51a includes data items, e.g., a patient ID 51aa, a family name of the patient Slab, a first name of the patient 51ac, a sex of the patient 51ad, and a birthday of the patient 51ae.

A filter button 51b is arranged at the first line in the patient list display area 51a, corresponding to the data item of the patient information record. The operation of the sort button 51c is similar to that of the sort button 41e of the schedule list screen 41 shown in Fig. 6.

The patient information records of all the patients are displayed on the patient list display area 51a by clicking an all-patient display button 51d on the right.

The patient list screen 51 includes at the bottom, an add button 51f which is clicked upon additionally registering the patient, an edit button 51g which edits the selected patient information record by selecting and clicking the patient information record in the patient list display area 51a, and an end button 51i for ending the patient list screen 51 by the click operation and for returning the patient list screen 51 to the called screen.

The patient list display area 51a displays the list of the new patient information registered from the in-hospital information system 5 connected to the endoscope image filing device 2 via the network, similarly to the display operation of the new patient information which is added by pressing the add button 51f.

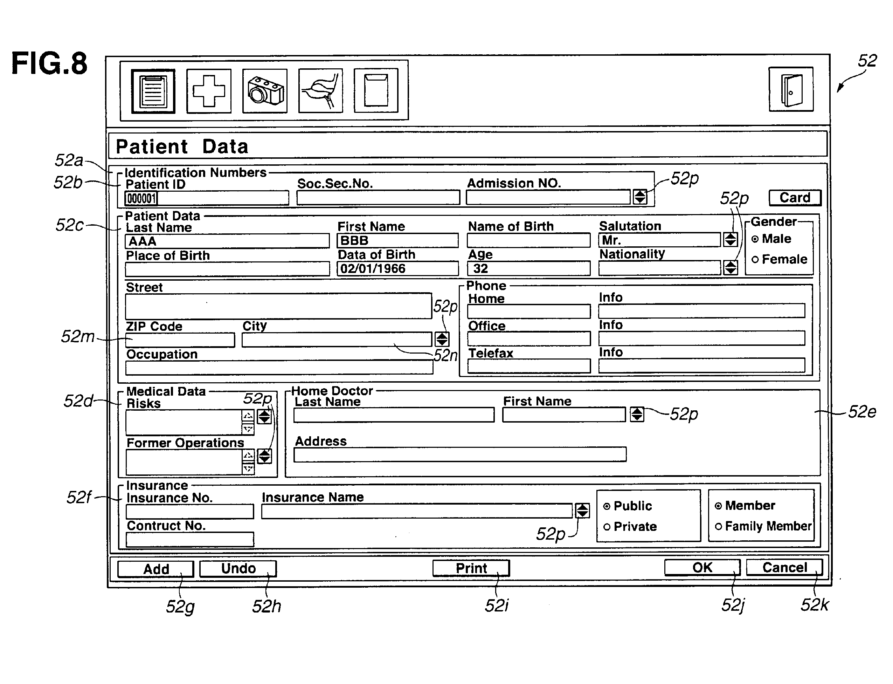

On the patient information editing screen 52 shown in Fig. 8, the contents of the patient information record to be registered additionally are inputted or the contents of the existing patient information record is edited.

An area 52a for editing the contents of the patient information record includes an area 52b for editing an ID No. such as the patient ID, an area 52c for editing a family name, birthday, birth place, sex, age, address, home phone No., office phone No., and office FAX No. of the patient, an area 52d for editing the outline of medical information such as a past treatment history of the patient, an area 52e for editing information on an attending physician, and an area 52f for editing insurance information.

The patient information editing screen 52 includes at the bottom, an add button 52g for newly registering the patient information record of the contents described in the area 52a by the click operation and for initializing the contents described in the area 52a to register a new next patient information record, a fresh start button 52h for returning the screen to the status in which the patient information editing screen 52 is opened and for editing the information again by the click operation, a print button 52i for printing out the contents of the patient information record by the click operation, an end button 52j for newly adding or updating the patient information record by the click operation, for closing the patient information editing screen 52, and for returning the screen to the patient list screen 51, and a stop button 52k for closing the patient information editing screen 52 by the click operation without newly adding and updating the patient information record.

The patient information record is registered by the click operation and the contents of the area 52a are initialized by using the add button 52g. Therefore, the operability is improved upon continuously registering new patient information of a plurality of patients.

A selecting list screen call button 52p is arranged at the right place adjacent to an input column 52m for inputting a zip code in the area 52c and an input column 52n for inputting a city name of the patient. The click operation of the selecting list screen call button 52p enables the operation for opening a selecting list screen (not shown) displaying the selecting list of the data inputted in the corresponding column, that is, the input columns 52m and 52n in this case. The operation for selecting the data to be inputted in the input columns 52m and 52n enables the operation for inputting the selected data in the input columns 52m and 52n.

The selecting list screen call button 52p arranged at another portion in the area 52a operates as mentioned above.

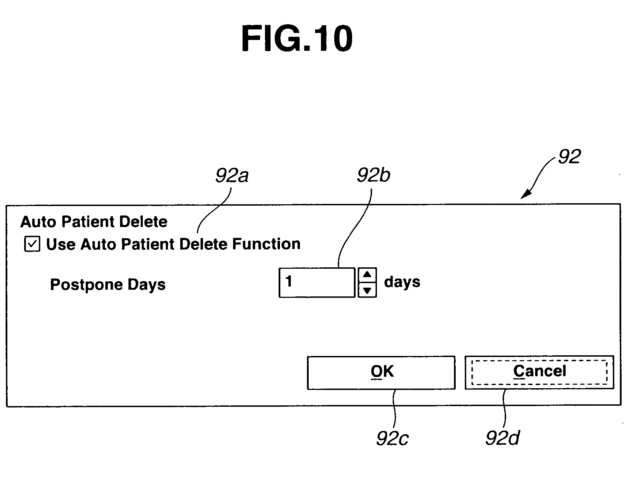

Next, a description is given with reference to Figs. 9 and 11 respecting the operation for deleting the patient information record, which is newly registered on the patient information list screen 51 or the patient information record which is newly registered from the in-house information system 5, but an endoscope examination is not executed on the patient for a time.

Referring to Fig. 9, in step S21, the system management screen 91 is opened. In step S22, the system management screen 91 is shifted and the patient information deletion setting screen 92 is opened.

In step S23, on the opened patient information deletion setting screen 92, it is inputted from the key board 23 whether or not the patient information record is deleted. In step S24, when the examination has not been performed for a time after newly registering the patient information, a set value of the number of days from the newly registered date (the number of days for no examination) is inputted and a condition for deleting the patient information is inputted. In step S25, the set value of the condition for deleting the patient information is recorded.

The set value is stored, for example, in the deleting condition storing area 32a of the hard disk 21e. That is, the hard disk 21e has the function of the deleting condition storing means for storing the deleting condition to delete the registered patient information. Of course, the registered patient information may be recorded on another recording medium or the like.

The set value may have a condition to delete only the patient information newly registered from the in-hospital information system 5, for which the examination is not performed for a predetermined term, or a condition to delete the patient information which is not registered to the patient history for a predetermined term. Alternatively, the set value may have a condition that the patient information is not updated after registration.

Fig. 10 shows a display example of the patient information deletion setting screen 92. The patient information deletion setting screen 92 includes a check area 92a for determining by using a check mark whether or not the patient information record is automatically deleted, and a setting area 92b for setting a term during which the examination is not performed after newly registering the patient information.

The set value is recorded on, e.g., the deleting condition storing area 32a in the hard disk 21e by clicking an OK button 92c on the patient information deletion setting screen 92. On the other hand, the set value is reset by clicking a cancel button 92d.

Next, a description is given respecting the operation flow for deleting the patient information with reference to Fig. 11.

Referring to Fig. 11, first, the image filing device 2 in the endoscope image filing system 3 is started. In step S31, the CPU 21a in the image filing device 2 reads the value which is previously set from the hard disk 21e. In step S32, the CPU 21a determines whether or not the set value corresponds to the deletion of the patient information. If it is determined that the set value does not correspond to the deletion of the patient information, the processing ends. Then, the log-in screen 31 for authenticating the operator shown in Fig. 4 is displayed.

On the contrary, if it is determined that the set value corresponds to the deletion of the patient information, in step S33, all the patient information, e.g., n pieces of patient information are obtained from the database in the endoscope image filing system 3. Subsequently, in step S34, a parameter j is set to 1 so as to check whether or not the patient information is to be deleted sequentially starting from the first patient information of the n pieces of patient information.

In step S35, the CPU 21a determines whether or not the number of lapse of days after registering the j-th patient information is over the number of days set in the patient information deletion setting screen 92 (N days in Fig. 11).

If the number of lapse of days is less than the set number of days, the j-th patient information should not be deleted and the processing routine shifts to step S39 whereupon the parameter j is incremented by 1, further, it is determined in step S40 whether or not the incremented (j+1)-th patient information reaches the n-th one and if not so, the processing routine shifts to step S35 whereupon the (j+1)-th patient information is subjected to the similar determining processing.

On the other hand, if the number of lapse of days is over the set number of days, in step S36, the CPU 21a determines whether or not the patient information indicates the patient who has already been examined by using the endoscope. If it is determined that the patient information indicates the patient who has already been examined by using the endoscope, the processing routine shifts to step S39 because the patient information should not be deleted.

If it is determined that patient information indicates the patient who has not been examined by using the endoscope, in step S37, the CPU 21a determines whether or not the patient history is registered.

If it is determined that the patient history is registered, the processing routine shifts to step S39 because the patient information should not be deleted.

On the other hand, if it is determined that the patient history is not registered, in step S38, the CPU 21a deletes the patient information as unnecessary one from the database. That is, the CPU 21a has the function of the patient information deleting means to delete the patient information which is registered in the database under the deleting condition based on the set value.

After that, for shifting the processing routine to the next patient information, in step S39, the parameter j is incremented by 1. In step S40, the CPU 21a determines whether or not all the n pieces of patient information are deleted by matching the parameter j to a condition of the (n+1)-th patient information. If the CPU 21a determines in step S40 that all the patient information are not processed, the processing routine returns to step S35 whereupon it is determined again whether or not the j-th patient information which is not determined is deleted.

As mentioned above, by determining whether or not the deletion is performed for all the patient information, the deleting processing of the patient information corresponding to the condition based on the set value ends.

On the patient information list screen 51, the deleted patient information is not displayed and therefore the search operability is improved for the operator.

According to the embodiment, the endoscope image filing system 3 is started and the operation flow for deleting the patient information is started. However, the operation flow for deleting the patient information may be started upon ending the endoscope image filing system 3 and, alternatively, may be started by user's setting. Or, the similar operation may be performed even in a status in which the image filing device 2 is started without connecting the endoscope device 1 to the endoscope image filing system 3.

Further, according to a modification of the embodiment as described below, the deletion of the patient information may be set.

That is, as mentioned above, the user who logs in the image filing device 2 or the endoscope image filing system 3 sets the patient information deletion setting screen 92 from the system management screen 91. The condition for setting the patient information from the database in the image filing device 2 is set. However, only the user having, e.g., a special authority of the system manager can set the patient information deletion setting screen 92 and unnecessary deletion of the patient information may be prevented.

A screen for logging in the patient information deletion setting screen 92 is displayed to set the patient information deletion setting screen 92 from the system management screen 91. Only the user having, e.g., a special authority of the system manager may log in the patient information deletion setting screen 92 by inputting the information on the ID code.

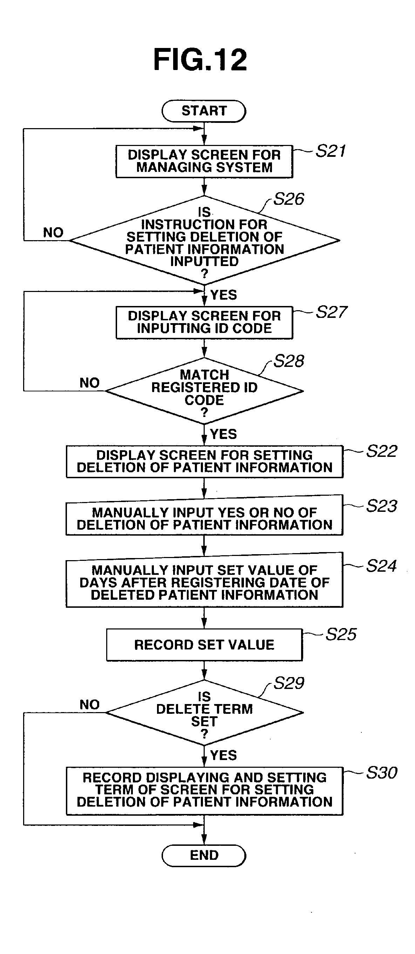

Fig. 12 shows the flowchart for the operation in the above-mentioned case. In other words, referring to Fig. 9, after displaying the system management screen 91 in step S21, in step S26, the CPU 21a determines whether or not an instruction for displaying the patient information deletion setting screen 92 is inputted. If the instruction is not in step S26, the processing routine returns to step S21. If the instruction is in step S26, in step S27, the CPU 21a displays a screen for inputting the ID code for the log-in of the patient information deletion setting screen 92.

If the ID code inputted from the key board 23 matches the ID code which has already been registered in the hard disk 21e or the like in step S28, the processing routine shifts to step S22. If the ID code inputted does not match the ID code registered in step S28, the processing routine returns to step S27 whereupon the screen for inputting ID code is displayed.

Through the above-mentioned processing, only the user having the special authority sets the patient information deletion setting screen 92 on which the patient information is deleted.

According to the modification of the embodiment, after recording the set value in step S25, in step S29, it is selected whether or not the deleting term for deleting the patient information is set.

If it is selected that the deleting term is set, in step S30, the patient information deleting term setting screen is displayed, the term for deleting the patient information is set on the patient information deleting term setting screen, the information on the deleting term is recorded on the hard disk 21e, and this processing ends. On the other hand, if it is selected that the deleting term is not set, the screen is set to default setting which is previously set (for example, the setting upon starting or ending the endoscope image filing system 3) and this processing ends.

General users browse the patient information deletion setting screen 92. However, the condition for setting the deletion of the patient information on the patient information deletion setting screen 92 may be changed or the like by inputting the information such as the ID code (which can be known only by permitted users).

Alternatively, the operation of the patient information deleting means (function) for setting the deletion of the patient information may be advantageous by inputting special information (which can be performed only by permitted users).

Having described the preferred embodiment of the invention referring to the accompanying drawings, it should be understood that the present invention is not limited to this precise embodiment and various changes and modifications thereof could be made by one skilled in the art without departing from the spirit or scope of the invention as defined in the appended claims.

## Claims

1. An endoscope image filing system **characterized by** comprising:
an image filing device for recording an endoscope image and forming a database in which patient information is registered;
registering means for registering the patient information;
deleting condition storing means for storing a deleting condition for deleting the patient information registered by said registering means; and
patient information deleting means for deleting the registered patient information based on the deleting condition stored by said deleting condition storing means.

2. The endoscope image filing system according to Claim 1, **characterized in that** said patient information deleting means comprises patient information deletion setting means for setting the deleting condition for deleting the patient information.

3. The endoscope image filing system according to Claim 1, **characterized in that** said system is connected to an endoscope device for executing an endoscope examination and generating the endoscope image.

4. The endoscope image filing system according to Claim 1, further **characterized by** comprising:
network connecting means for connection via a network.

5. The endoscope image filing system according to Claim 1, **characterized in that** said patient information deleting means comprises deleting term setting means for setting a deleting term for which the patient information is deleted upon starting said system.

6. The endoscope image filing system according to Claim 1, **characterized in that** said patient information deleting means comprises automatic deletion selecting means for selecting whether the patient information is automatically deleted.

7. The endoscope image filing system according to Claim 2, **characterized in that** said patient information deletion setting means comprises deleting term setting means for setting a term between the registration of the patient information and a deleting timing.

8. The endoscope image filing system according to Claim 7, **characterized in that** said patient information deletion setting means sets to the deleting condition, the presence or absence of updated information obtained by changing the patient information within the term.

9. The endoscope image filing system according to Claim 7, **characterized in that** the update information indicates whether or not the endoscope examination is performed.

10. The endoscope image filing system according to Claim 1, **characterized in that** an authenticating screen for authenticating the operator is displayed upon starting said system.

11. The endoscope image filing system according to Claim 1, **characterized in that** after authenticating the operator upon starting said system, a schedule screen indicating a schedule of the endoscope examination is displayed.

12. The endoscope image filing system according to Claim 1, **characterized in that** a schedule screen indicating a schedule for the endoscope examination is displayed after starting said system, and a plurality of screens such as a patient information list screen for displaying a list of the patient information can selectively be shifted and be displayed from the schedule screen.

13. The endoscope image filing system according to Claim 12, **characterized in that** the screen can be changed to a system management screen for managing. said system from the schedule screen.

14. The endoscope image filing system according to Claim 13, **characterized in that** the screen can be changed to a patient information deleting condition setting screen for setting a condition for deleting the patient information from the system management screen.

15. The endoscope image filing system according to Claim 12, **characterized in that** the screen can be changed to a patient information editing screen for registering the patient information from the patient information list display screen.

16. The endoscope image filing system according to Claim 1, **characterized in that** authenticating information needs to be inputted to operate substantially the patient information deleting means for deleting the registered patient information.
